(19) **Europäisches Patentamt / European Patent Office / Office européen des brevets**

(11) **EP 4 534 021 A1**

(12) **EUROPEAN PATENT APPLICATION**
published in accordance with Art. 153(4) EPC

(43) Date of publication:
**09.04.2025 Bulletin 2025/15**

(21) Application number: **22944944.2**

(22) Date of filing: **03.06.2022**

(51) International Patent Classification (IPC):
**A61B 10/00** (2006.01)

(52) Cooperative Patent Classification (CPC):
**A61B 10/00; G10L 25/66**

(86) International application number:
**PCT/JP2022/022687**

(87) International publication number:
**WO 2023/233667 (07.12.2023 Gazette 2023/49)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicants:
• **PST Inc.**
  **Yokohama-shi, Kanagawa 231-0023 (JP)**
• **Public University Corporation Yokohama City University**
  **Yokohama-shi, Kanagawa 236-0027 (JP)**

(72) Inventors:
• **OMIYA, Yasuhiro**
  **Yokohama-shi, Kanagawa 231-0023 (JP)**
• **TAKANO, Takeshi**
  **Yokohama-shi, Kanagawa 231-0023 (JP)**
• **ENDO, Koji**
  **Yokohama-shi, Kanagawa 231-0023 (JP)**
• **OKADA, Kozo**
  **Yokohama-shi Kanagawa 232-0024 (JP)**
• **KOBAYASHI, Yusuke**
  **Yokohama-shi, Kanagawa 236-0004 (JP)**

(74) Representative: **Berggren Oy**
**P.O. Box 16**
**Eteläinen Rautatiekatu 10A**
**00101 Helsinki (FI)**

(54) **INFORMATION PROCESSING DEVICE, INFORMATION PROCESSING METHOD, INFORMATION PROCESSING SYSTEM, AND INFORMATION PROCESSING PROGRAM**

(57)   An information processing device acquires speech data that is time series data of speech spoken by a user. Based on the speech data, the information processing device computes state information that represents a cardiac condition of the user, and the information processing device outputs the computed state information.

FIG.1

EP 4 534 021 A1

**Description**

TECHNICAL FIELD

The technology of this disclosure relates to an information processing device, an information processing method, an information processing system and an information processing program.

BACKGROUND ART

International Patent Publication No. 2020/013296 discloses a device that estimates a mental disease or a neurological disease. This device computes various acoustic parameters from speech data of a user, and uses the acoustic parameters to estimate whether or not the user has the mental disease or neurological disease.

SUMMARY OF INVENTION

TECHNICAL PROBLEM

The device disclosed in International Patent Publication No. 2020/013296 uses acoustic parameters computed from speech data to estimate a mental disease or neurological disease.
However, various kinds of information are included in speech spoken by a user, and it may be possible not only to estimate a mental disease or neurological disease from speech but also to estimate other diseases affecting the user.
The technology of this disclosure is made in consideration of the circumstances described above and provides an information processing device, an information processing method, an information processing system and an information processing program that may estimate a cardiac condition of a user from speech data that is time series data of speech spoken by the user.

SOLUTION TO PROBLEM

A first aspect of the present disclosure for achieving the object described above is an information processing device including: an acquisition section that acquires speech data that is time series data of speech spoken by a user; a computing section that computes state information representing a cardiac condition of the user based on the speech data acquired by the acquisition section; and an output section that outputs the state information computed by the computing section.
A second aspect of the present disclosure is an information processing method including causing a computer to execute processing including: acquiring speech data that is time series data of speech spoken by a user; computing state information representing a cardiac condition of the user based on the acquired speech data; and outputting the computed state information.
A third aspect of the present disclosure is an information processing program for causing a computer to execute processing including: acquiring speech data that is time series data of speech spoken by a user; computing state information representing a cardiac condition of the user based on the acquired speech data; and outputting the computed state information.

ADVANTAGEOUS EFFECTS OF INVENTION

According to the technology of the disclosure, an effect is provided in that a cardiac condition of a user may be estimated from speech data that is time series data of speech spoken by the user.

BRIEF DESCRIPTION OF DRAWINGS

Fig. 1 is a diagram showing an example of schematic structures of an information processing system according to a first exemplary embodiment.
Fig. 2A, Fig. 2B and Fig. 2C are diagrams for describing speech features used in the present exemplary embodiment.
Fig. 3 is a diagram (a magnified diagram of area P1 in Fig. 2C) for describing a speech characteristic quantity used in the present exemplary embodiment.
Fig. 4 is a diagram schematically showing an example of a usage situation of the information processing system according to the first exemplary embodiment.

Fig. 5 is a diagram showing an example of a computer constituting an information processing device.

Fig. 6 is a flowchart showing an example of processing executed by the information processing device according to the first exemplary embodiment.

Fig. 7 is a diagram showing an example of schematic structures of an information processing system according to a second exemplary embodiment.

Fig. 8 is a diagram schematically showing an example of a usage situation of the information processing system according to the second exemplary embodiment.

Fig. 9 is a diagram schematically showing another example of a usage situation of the information processing system according to the second exemplary embodiment.

Fig. 10 is a diagram for explaining speech features described in an Example.

Fig. 11 is a diagram for describing an Example.

DETAILED DESCRIPTION

Below, exemplary embodiments of the present disclosure are described in detail with reference to the attached drawings.

= Information processing system according to first exemplary embodiment =

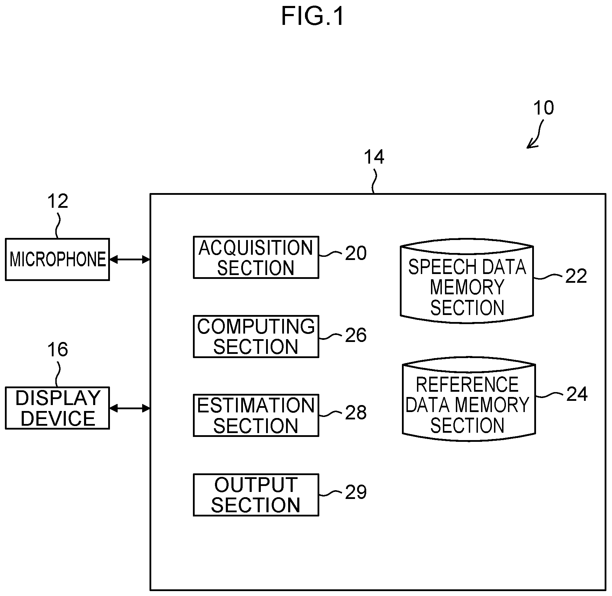

Fig. 1 illustrates an information processing system 10 according to a first exemplary embodiment. As shown in Fig. 1, the information processing system 10 according to the first exemplary embodiment is provided with a microphone 12, an information processing device 14 and a display device 16.

The information processing system 10 may estimate a cardiac condition of a user based on speech from the user that is collected by the microphone 12. The present exemplary embodiment describes an example in which, as the cardiac condition of the user, the information processing system 10 computes a degree of heart failure of the user and estimates whether or not the user has heart failure based on this degree. The cardiac condition of the user is not limited to a degree of heart failure of the user but may be a cardiac load condition of the user, a pulmonary congestion condition of the user, a fluid retention condition of the user or the like. The degree of heart failure of the user is an example of state information representing a cardiac condition of a user.

The information processing device 14 of the information processing system 10 according to the first exemplary embodiment generates an envelope of speech data, which is time series data of speech spoken by the user, and applies a Fourier transform to the envelope. For respective combinations of one frequency section (below referred to simply as a first frequency section) and a frequency section adjacent to the first frequency section (below referred to simply as a second frequency section) in an analysis object frequency section of the Fourier transform result, the information processing device 14 computes differences between spectral powers of the first frequency sections and spectral powers of the second frequency sections. The information processing device 14 computes an integration result in which the differences in the analysis object frequency section are integrated, and sets the integration result as a single speech characteristic quantity. In the present exemplary embodiment, the speech characteristic quantity is referred to as a voice modulation index (VMI). The information processing device 14 then computes a degree of heart failure of the user based on the VMI. This is described more specifically below.

As shown in Fig. 1, functionally, the information processing device 14 includes an acquisition section 20, a speech data memory section 22, a reference data memory section 24, a computing section 26, an estimation section 28 and an output section 29. The information processing device 14 is realized by a computer as described below.

The acquisition section 20 acquires speech data, which is time series data of speech spoken by a user. The user is a subject for whom the presence or absence of heart failure is to be estimated. The acquisition section 20 stores the speech data to the speech data memory section 22.

The speech data memory section 22 stores the speech data acquired by the acquisition section 20.

The reference data memory section 24 stores speech data (referred to below simply as reference data) of reference users who are already known to have or not have heart failure. The reference data is speech data spoken by people who have been diagnosed with heart failure and speech data spoken by people who have been diagnosed as not having heart failure. The reference data memory section 24 may store derived data based on the speech data. For example, the reference data memory section 24 may store speech features extracted from the reference data.

The reference data memory section 24 stores a computation model for using one or more speech features extracted from the speech data to compute the degree of heart failure of the user. This computation model is, for example, a statistical model or a machine learning model. For example, when a regression model is used as a statistical model, regression equations of the regression model and coefficient values of the regression model are stored in the reference data memory section 24 as the computation model. As another example, when a machine learning model is employed, the machine learning model that is a combination of structural formulas and learned parameter values of the machine learning model is stored in the reference data memory section 24 as the computation model. The statistical model or machine learning model acquires the coefficients or parameters in advance based on training data collected in advance. These computation models are to be used when computing degrees of heart failure of users.

Rather than employing a statistical model, a machine learning model or the like, a degree of similarity between speech data obtained from the user or speech features extracted from the speech data and reference data or speech features extracted from the reference data may be used to compute the degree of heart failure of the user. The present exemplary embodiment describes an example in which a computation model that is for computing degrees of heart failure of users is used with speech features to compute the degree of heart failure of a user.

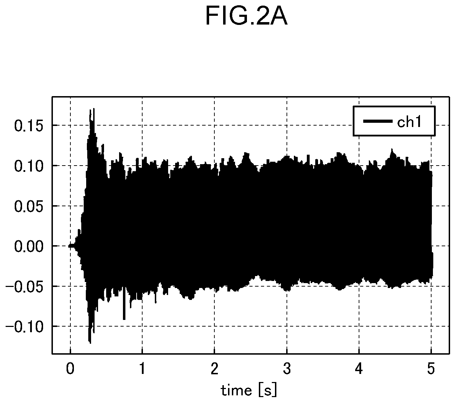

The computing section 26 reads speech data memorized at the speech data memory section 22. The computing section 26 executes various kind of processing on the speech data and estimates the degree of heart failure of the user based on the obtained results. A method of generating the VMI, which is one speech characteristic quantity used in the present exemplary embodiment, is specifically described below. Fig. 2A to Fig. 2C show diagrams for describing the VMI.

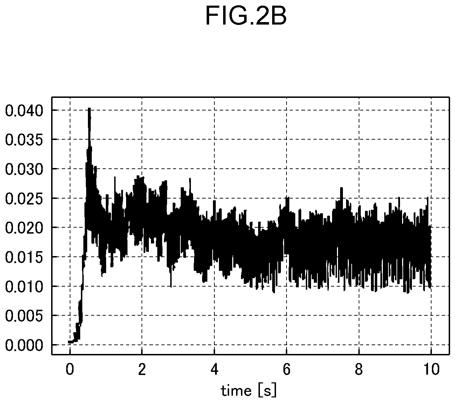

Fig. 2A is a diagram showing an example of speech data. The computing section 26 uses previously known methods to generate an envelope as illustrated in Fig. 2B from speech data as illustrated in Fig. 2A. The vertical axes of Fig. 2A and Fig. 2B represent amplitudes (or acoustic pressures) of the speech data.

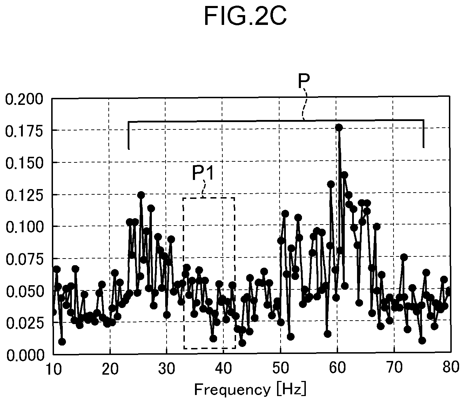

Then, the computing section 26 applies a Fourier transform to the envelope as illustrated in Fig. 2B, obtaining a Fourier transform result of the envelope as illustrated in Fig. 2C. The vertical axis in Fig. 2C represents spectral power.

Next, for respective combinations of a first frequency section and a second frequency section adjacent to the first frequency section in an analysis object frequency section P of the Fourier transform result as illustrated in Fig. 2C, the computing section 26 computes differences between the spectral powers of first frequency values and the spectral powers of second frequency values. As an example here, the analysis object frequency section P is specified with a minimum frequency of 25 Hz and a maximum frequency of 75 Hz.

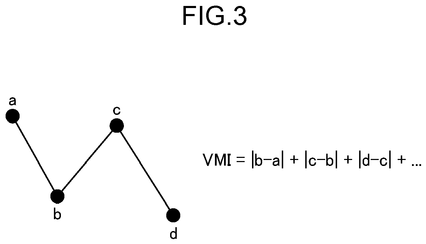

Fig. 3 is a magnified diagram of an area P1 in Fig. 2C. More specifically, the computing section 26 specifies a first frequency value and a second frequency value adjacent to the first frequency value in the frequency section P1 within the analysis object frequency section P, as illustrated in Fig. 3. The computing section 26 computes a difference between a spectral power a at the first frequency value and a spectral power b at the second frequency value as illustrated in Fig. 3.

Similarly, the computing section 26 computes a difference between the spectral power b and a spectral power c as illustrated in Fig. 3. The computing section 26 also computes a difference between the spectral power c and a spectral power d as illustrated in Fig. 3.

The computing section 26 computes an integration result in which sums of the above-described differences computed in the analysis object frequency section P are integrated, and sets the integration result as the VMI that is a single speech characteristic quantity.

Now, the VMI proposed for the present exemplary embodiment is described. It is thought that when a cardiac condition of a user is poor, for example, a heart failure condition, water accumulates in the lungs, and this manifests in the voice. In this condition, for example, phlegm is more likely to occur in the throat of the user, and there is a strong tendency for the voice of the user to sound raspy.

Rasping in the voice of the user is thought to correspond with a frequency region from 25 to 75 Hz. The greater spectral changes in the speech data are ("sawtoothing" of the waveform), the stronger the rasping of the actual voice.

For the VMI proposed in the present exemplary embodiment, differences between spectral power of one frequency in the speech data and the spectral power adjacent to that spectral power are computed and the differences are integrated. Consequently, the VMI can be said to be a characteristic quantity that detects raspiness in the voice of the user, and can be said to be a speech characteristic quantity that enables accurate detection of a cardiac condition of the user.

The computing section 26 extracts plural other speech features from the speech data. For example, the computing section 26 extracts a harmonics-to-noise ratio (HNR) and a continuous vocalization duration of sustained vowel sounds from the speech data as speech features. The HNR is, for example, the characteristic quantity disclosed in the below Reference Document 1.

Reference Document 1; "Harmonic to Noise Ratio Measurement - Selection of Window and Length", Procedia Computer Science, Volume 138, 2018, Pages 280-285.

The computing section 26 further extracts various speech features as disclosed in International Patent Publication No. 2020/013296 from the speech data. The computing section 26 may also obtain a spectrogram from the speech data and extract features from the spectrogram.

Based on plural speech features as described above, the computing section 26 computes a score representing a degree of heart failure of the user. The score representing the degree of heart failure of the user according to the present exemplary embodiment may indicate a level of probability that the user has heart failure. More specifically, the computing section 26 reads reference data stored in the reference data memory section 24, and the computing section 26 extracts the same plural speech features from the reference data. The computing section 26 computes the score representing the degree of heart failure of the user based on the plural speech features extracted from the speech data of the user and the plural speech features extracted from the reference data. Relationships of the score may be specified in advance such that, for example, the greater the value of the score, the higher the probability that the user has heart failure, and the smaller the value of the score, the lower the probability of heart failure. Alternatively, relationships of the score may be specified in advance such that, for example, the smaller the value of the score, the higher the probability that the user has heart failure, and the greater the value of the score, the lower the probability of heart failure.

For example, a statistical model of degree of heart failure uses plural speech features extracted from the reference data acquired from people who have been diagnosed with heart failure, and the computing section 26 uses this statistical model to compute a score representing the degree of heart failure of the user whose score is being calculated from the speech data of the user.

Based on the score computed by the computing section 26, the estimation section 28 estimates whether or not the user has heart failure. For example, when the score is at least a predetermined threshold, the estimation section 28 estimates that the user has heart failure, and when the score is less than the predetermined threshold, the estimation section 28 estimates that the user does not have heart failure.

The output section 29 outputs the estimation result estimated by the estimation section 28. The output section 29 may output the score representing the degree of heart failure itself as the estimation result.

The display device 16 displays the estimation result outputted from the estimation section 28.

A clinical practitioner operating the information processing device 14 or the user checks the estimation result outputted from the display device 16 and checks the possibility that the user has heart failure.

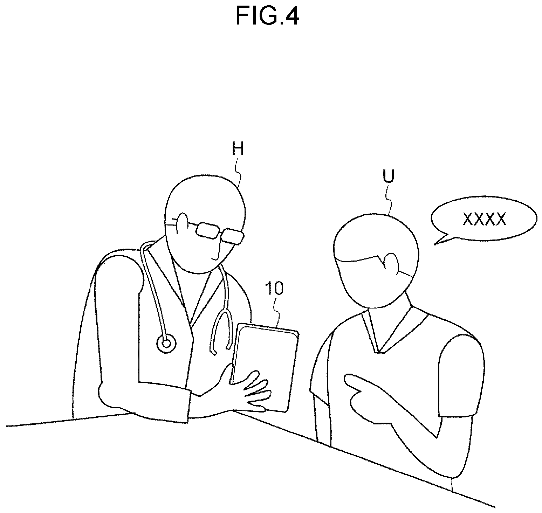

The information processing system 10 according to the present exemplary embodiment is expected to be used, for example, under conditions as illustrated in Fig. 4.

In the example in Fig. 4, a clinical practitioner H such as a doctor or the like holds a tablet terminal, which is an example of the information processing system 10. The clinical practitioner H uses a microphone (not shown in the drawing) provided at the tablet terminal to collect speech data from a user U, who is an examination subject. Based on the speech data of the user U, the tablet terminal estimates whether or not the user U has heart failure and outputs an estimation result to a display unit (not shown in the drawing). The clinical practitioner H refers to the estimation result displayed at the display unit (not shown in the drawing) of the tablet terminal and the clinical practitioner H judges the degree of heart failure of the user U.

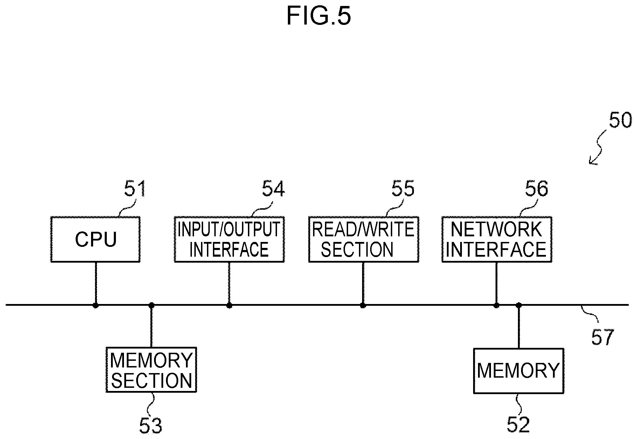

The information processing device 14 may be realized by, for example, a computer 50 illustrated in Fig. 5. The computer 50 is provided with a CPU 51, a memory 52 that serves as a temporary memory region, and a nonvolatile memory section 53. The computer 50 is further provided with an input/output interface (I/F) 54 to which external equipment, output devices and the like are connected, and a read/write (R/W) section 55 that controls reading and writing of data at a recording medium. The computer 50 is also provided with a network interface 56 that is connected to a network such as the Internet or the like. The CPU 51, memory 52, memory section 53, input/output interface 54, read/write section 55 and network interface 56 are connected to one another via a bus 57.

The memory section 53 may be realized by a hard disk drive (HDD), solid-state drive (SSD), flash memory or the like. A program for causing functioning of the computer 50 is memorized at the memory section 53, which serves as a memory medium. The CPU 51 reads the program from the memory section 53, loads the program into the memory 52, and sequentially executes processes of the program.

- Operation of the information processing system according to the first exemplary embodiment -

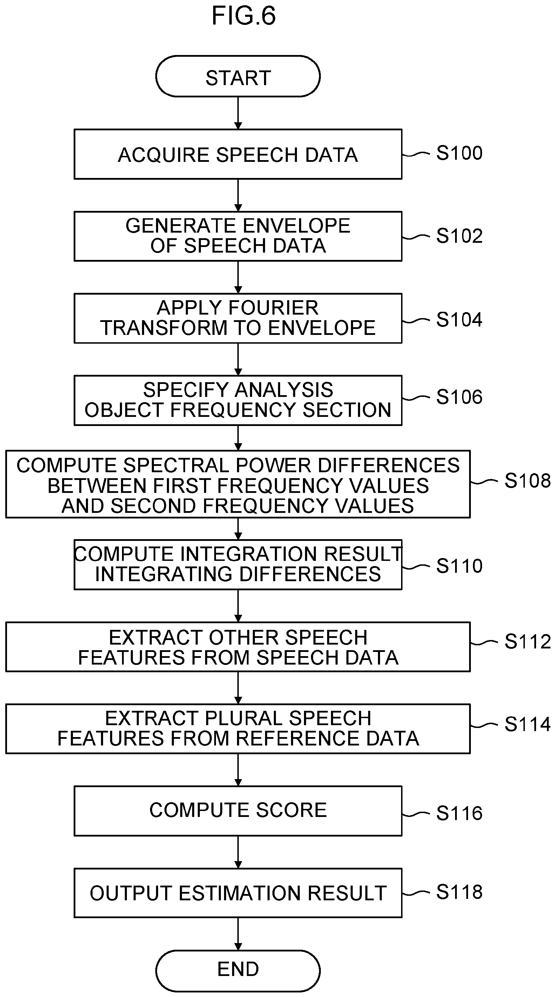

Now, specific operations of the information processing system 10 according to the first exemplary embodiment are described. The information processing device 14 of the information processing system 10 executes the processing shown in Fig. 6.

First, in step S100, the acquisition section 20 acquires speech data of the user that is collected by the microphone 12. The acquisition section 20 stores the speech data to the speech data memory section 22.

In step S102, the computing section 26 reads the speech data stored to the speech data memory section in step S100, and generates an envelope such as that illustrated in Fig. 2B from the speech data.

In step S104, the computing section 26 applies a Fourier transform to the envelope generated in step S102, thus acquiring a Fourier transform result of the envelope such as that illustrated in Fig. 2C.

In step S106, the computing section 26 specifies an analysis object frequency section P such as that illustrated in Fig. 2C for the Fourier transform result acquired in step S104.

In step S108, for respective combinations of a first frequency value and a second frequency value adjacent to the first frequency value in the analysis object frequency section P of the Fourier transform result specified in step S106, the computing section 26 computes differences between the spectral powers of the first frequency values and the spectral powers of the second frequency values.

In step S110, the computing section 26 computes an integration result in which sums of the differences computed in step S108 are integrated, and sets the integration result as a single speech characteristic quantity.

In step S112, the computing section 26 computes plural other speech features from the speech data acquired in step S100.

In step S114, the computing section 26 reads reference data from the reference data memory section 24 and may extract the speech characteristic quantity as computed in step S110 and the plural speech features as extracted in step S112 from the reference data.

In step S116, the computing section 26 computes a score representing a degree of heart failure of the user speaking the speech data acquired in step S110 based on the plural speech features of the speech data acquired in step S110 and step S112, the plural speech features of the reference data extracted in step S114, and a statistical model stored at the reference data memory section 24. More specifically, the computing section 26 inputs the plural speech features into the statistical model and the computing section 26 uses a value outputted from the statistical model as the score representing the degree of heart failure of the user.

In step S118, the estimation section 28 estimates whether or not the user has heart failure based on the score computed in step S116 described above. For example, when the score is at least the predetermined threshold, the estimation section 28 estimates that the user has heart failure, and when the score is less than the predetermined threshold, the estimation section 28 estimates that the user does not have heart failure. The estimation section 28 also outputs the estimation result in step S118.

The output section 29 outputs the estimation result from the estimation section 28. The display device 16 displays the estimation result outputted from the output section 29. A clinical practitioner or user operating the information processing device 14 checks the estimation result outputted from the display device 16 and the clinical practitioner or user checks the degree of heart failure.

As described above, the information processing device 14 of the information processing system 10 according to the first exemplary embodiment computes a degree of heart failure of a user based on speech data that is time series data of speech spoken by the user, and outputs the degree that is computed. Therefore, a clinical practitioner or user may estimate a degree of heart failure from the speech data that is time series data of speech spoken by the user. The clinical practitioner H in Fig. 4 may be replaced with a smart home appliance, a smart speaker, an avatar or the like.

The information processing device 14 generates an envelope of speech data and applies a Fourier transform to the envelope, thus acquiring a Fourier transform result of the envelope. For respective combinations of a first frequency value and a second frequency value adjacent to the first frequency value in the analysis object frequency section of the Fourier transform result, the information processing device 14 computes differences between spectral powers of the first frequency values and spectral powers of the second frequency values. The information processing device 14 computes an integration result integrating the differences in the analysis object frequency section, sets the integration result as a speech characteristic quantity, and computes a degree of heart failure of a user based on this speech characteristic quantity. Speech features used when computing a degree of heart failure of a user may include one or more of an HNR, a continuous vocalization duration, pause durations between utterances as a proportion of the time taken to speak plural utterances, a length of pause times between utterances, a length of time taken for an utterance, and a speaking speed. As a result, the degree of heart failure of the user may be estimated accurately.

= Information processing system according to second exemplary embodiment =

Now, a second exemplary embodiment is described. Structures of an information processing system according to the second exemplary embodiment that are the same as in the first exemplary embodiment are assigned the same reference symbols and are not described here.

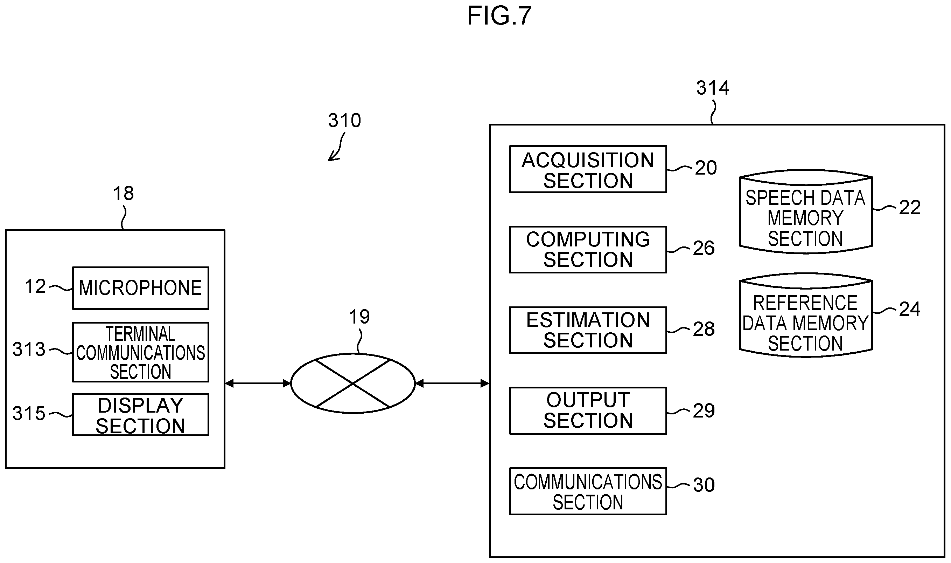

Fig. 7 illustrates an information processing system 310 according to the second exemplary embodiment. As shown in Fig. 7, the information processing system 310 is provided with a user terminal 18 and an information processing device 314. The information processing device 314 is additionally provided with a communications section 30.

The information processing device 314 of the information processing system 310 estimates a degree of heart failure of a user based on speech of the user collected by the microphone 12, which is provided at the user terminal 18.

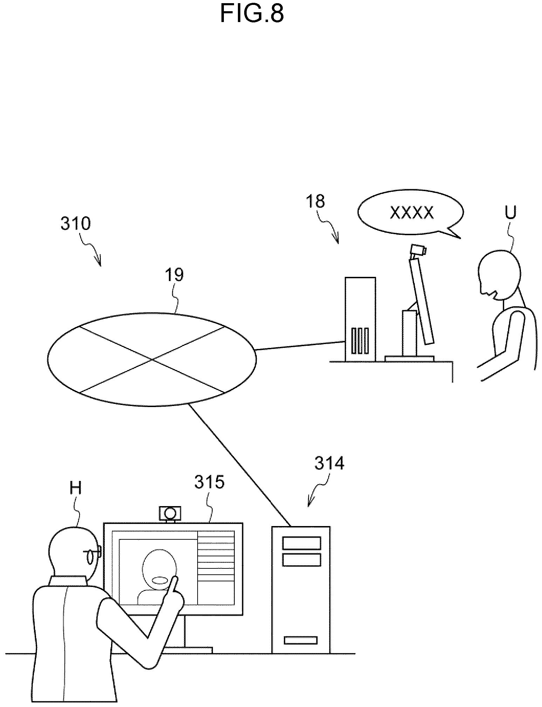

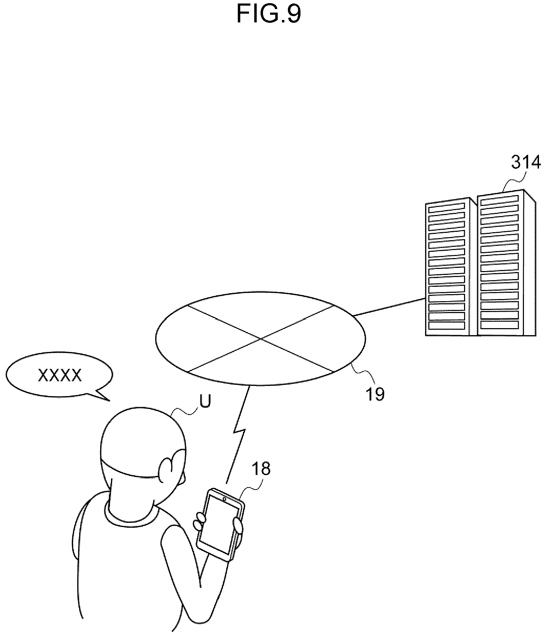

The information processing system 310 according to the second exemplary embodiment is expected to be used, for example, under conditions as illustrated in Fig. 8 and Fig. 9.

In the example in Fig. 8, a clinical practitioner H in a hospital or the like operates the information processing device 314, and a user U who is the examination subject operates the user terminal 18. The user U collects their own speech data with the microphone 12 of the user terminal 18 being operated by the user U. The user terminal 18 transmits the speech data to the information processing device 314 via a network 19, such as the Internet or the like.

The information processing device 314 receives the speech data of the user U transmitted from the user terminal 18. Based on the received speech data, the information processing device 314 estimates a degree of heart failure of the user U, and outputs an estimation result to a display section 315 of the information processing device 314. The clinical practitioner H refers to the estimation result displayed at the display section 315 of the information processing device 314 and judges the degree of heart failure of the user U.

In the example in Fig. 9, a user U who is the examination subject collects their own speech data with the microphone 12 of the user terminal 18 being operated by the user U. The user terminal 18 transmits the speech data to the information processing device 314 via the network 19, such as the Internet or the like. The information processing device 314 receives the speech data of the user U transmitted from the user terminal 18. Based on the received speech data, the information processing device 314 estimates a degree of heart failure of the user U, and transmits an estimation result to the user terminal 18. The user terminal 18 receives the estimation result transmitted from the information processing device 314 and displays the estimation result at a display section (not shown in the drawings). The user checks the estimation result and checks their own degree of heart failure.

The information processing device 314 executes an information processing routine similar to Fig. 6 described above.

As described above, the information processing system according to the second exemplary embodiment may use the information processing device 314 that is located in the Cloud to estimate a degree of heart failure of a user.

Utilizing the information processing system according to the second exemplary embodiment enables estimations of heart failure of users even outside the hospital. There are many advantages in enabling estimation of heart failure outside the hospital, providing great social value. For example, once a patient has been diagnosed with heart failure, after the patient is discharged from the hospital, there is a high likelihood of the heart failure worsening or recurring outside the hospital and the patient being repeatedly re-admitted. **In** this kind of situation, if symptoms of the heart failure worsening can be detected at as early a stage as possible and prompt measures can be taken, there is hope that re-admission due to the heart failure worsening can be prevented, and there is a high likelihood that the patient may recover quickly in spite of the worsening heart failure. Furthermore, when examination in the hospital is difficult because of a disaster, infectious disease epidemic or the like, this technology may enable adaptations for early detection and management of heart failure, which is a serious illness. Discovering heart failure may require monitoring of states including blood pressure and pulse of the patient, and also blood sampling, X-ray examinations and the like. Continuous monitoring of these states for a user outside the hospital is difficult.

In contrast, according to the information processing system of the present exemplary embodiment, a degree of heart failure of a user may be computed based on speech data of the user. Therefore, for example, even a user at home may check their degree of heart failure. By utilizing the information processing system according to the present exemplary embodiment, doctors other than doctors specializing in the circulatory system and other clinical staff or care staff may judge heart failure of users. Therefore, a change in degree of heart failure of a patient may be detected promptly.

= Example 1 =

Now, Example 1 is described. Example 1 illustrates experimental results relating to applicability of the present exemplary embodiment to the included speech features: the voice modulation index (VMI); the harmonics-to-noise ratio (HNR); the continuous vocalization duration; pause durations between utterances as a proportion of the time taken to speak plural utterances; the length of pause times between utterances; the length of time taken for an utterance; and the speaking speed. In the present Example 1, phrases as shown in the tables were spoken by examination subjects, correlations between speech features obtained from the speech data and heart failure indicators of examination subjects were computed, and accuracies of judging whether or not the examination subjects had heart failure conditions were computed.

The indicators in Table 2 and below of the present Example are the indicators shown in Table 1.

Table 1

| NYHA | New York Heart Association (NYHA) functional classifications<br>I: Presence of cardiac disease. No symptoms in ordinary physical activity.<br>II: Ordinary physical activity (such as climbing a slope or steps) causes symptoms.<br>III: Less than ordinary physical activity (such as walking on the flat) causes symptoms.<br>IV: Symptoms of heart failure and angina even at rest. |
| --- | --- |
| BNP | Brain natriuretic peptide: A hormone secreted from the heart in greater amounts when cardiac loads are greater |
| diff_BNP | Differential relative to maximum value of BNP in the same subject |
| weight | Body weight |
| diff_weight | Differential relative to maximum body weight of the same subject |

Table 2 below shows results of calculating correlations between a conventionally known speech characteristic quantity, the zero-crossing rate (ZCR), or the VMI and the various indicators used when judging degrees of heart failure.

- Analysis of exemplary phrases and the sustained vowel "/a:/" -

Table 2

| Correlations with speech indicators (longitudinal data from 23 cases: speech data nos. 357–1244) | Spoken phrases | | | | | |
|---|---|---|---|---|---|---|
| | "I-ro-ha-ni-ho-he-to" | | "Kokoro ga odayaka desu" | | "/a:/" | |
| | ZCR | VMI | ZCR | VMI | ZCR | VMI |
| NYHA | −0.0060 | 0.2013 | −0.0891 | 0.2706 | −0.0983 | −0.2409 |
| BNP | 0.1954 | 0.0438 | 0.1916 | −0.0533 | 0.1094 | −0.1515 |
| diff_BNP | −0.0882 | 0.1885 | 0.0897 | −0.0380 | −0.0170 | 0.4591 |
| weight | −0.0433 | −0.1145 | 0.0289 | 0.0700 | 0.1419 | 0.0202 |
| diff_weight | 0.0822 | 0.1751 | 0.0219 | 0.0004 | 0.1510 | −0.1356 |

From the comparisons in Table 2 above between VMI, the speech characteristic quantity used in the exemplary embodiment, and ZCR, the conventionally known speech characteristic quantity, it can be seen that the correlations of NYHA with VMI tended to be greater than the correlations with ZCR.

Therefore, it can be seen that a speech characteristic quantity used in the exemplary embodiment, VMI, is a useful speech characteristic quantity for estimating degrees of heart failure.

Table 3 shows results of calculating correlations between various speech features including the HNR and the various indicators used when judging levels of heart failure.

- Analysis of the sustained vowel "/a:/" -

Table 3

| Correlations with speech indicators (longitudinal data from 23 cases: speech data no. 755) | Shimmer | Jitter | HNR |
|---|---|---|---|
| NYHA | −0.0268 | −0.0140 | −0.0192 |
| BNP | 0.4765 | 0.4522 | −0.4812 |
| diff_BNP | −0.3231 | −0.2547 | 0.3632 |
| weight | −0.1594 | −0.1297 | 0.1763 |
| diff_weight | 0.2659 | 0.2660 | −0.3145 |

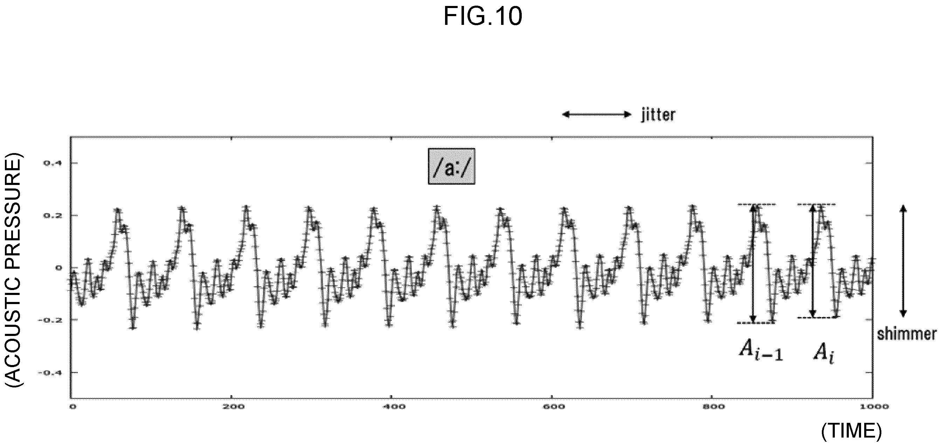

Fig. 10 shows a graph for explaining "Shimmer" and "Jitter" in Table 3 above. Shimmer and jitter are voice features disclosed in International Patent Publication No. 2020/013296. The subscript i in expression 1 below is an index for distinguishing individual waves in a periodically repeated signal. The symbol N represents a total number of periodic repetitions of the signal. The symbol T in the expression below represents the period, and the symbol A represents amplitude.

$$Jitter = \frac{\sum_{i=1}^{N-1} \frac{1}{N-1} |T_i - T_{i-1}|}{\overline{T_i}}$$

$$Shimmer = \frac{\sum_{i=1}^{N-1} \frac{1}{N-1} |A_i - A_{i-1}|}{\overline{A_i}}$$

The HNR represents an energy ratio between noise components and harmonic components. As shown in Table 3 above, it can be seen that correlations between the speech characteristic quantity HNR and the various indicators tend to be greater than correlations between the conventionally known speech features shimmer, jitter and ZCR and the various indicators. Therefore, it can be seen that HNR, a speech characteristic quantity utilized in the exemplary embodiment, is a useful speech characteristic quantity for estimating heart failure.

Table 4 shows results of computing correlations between speech features-the continuous vocalization duration of sustained vowel sounds, pause durations between utterances as a proportion of the time taken to speak plural utterances (below referred to as the pause proportion), the length of pause times between utterances (below referred to as the pause length), the length of time taken for an utterance (below referred to as the utterance length), and the speaking speed-and the various indicators used when judging degrees of heart failure.

- Analysis of the sustained vowel "/a:/" -

Table 4

| Correlations with speech indicators (longitudinal data from 23 cases: speech data nos. 344–755) | Sustained vowel "/a:/" | Speech data of plural standard utterances and pauses: "I-ro-ha-ni-ho-he-to", "kokoro ga odayaka desu", etc. | | | |
| --- | --- | --- | --- | --- | --- |
| | Continuous vocalization duration | Pause proportion | Speaking speed | Utterance length | Pause length |
| NYHA | −0.4094 | 0.0282 | 0.2506 | 0.3485 | 0.2924 |
| BNP | −0.3844 | 0.0906 | 0.2198 | 0.2683 | 0.2186 |
| diff_BNP | 0.3270 | 0.0060 | −0.2933 | −0.0870 | −0.0479 |
| weight | 0.3383 | 0.4475 | −0.2589 | −0.1898 | 0.2438 |
| diff_weight | −0.3265 | 0.1634 | 0.1464 | 0.1832 | 0.2466 |

As shown in Table 4 above, it can be seen that correlations between the continuous vocalization duration of sustained vowel sounds, pause durations between utterances as a proportion of the time taken to speak plural utterances, the length of pause times between utterances, the length of time taken for an utterance, and the speaking speed and the various indicators tend to be greater than correlations between the conventionally known speech characteristic quantity ZCR and the various indicators. Therefore, it can be seen that the continuous vocalization duration of sustained vowel sounds, the pause durations between utterances as a proportion of the time taken to speak plural utterances, the length of pause times between utterances, the length of time taken for an utterance, and the speaking speed are useful speech features for estimating degrees of heart failure.

- Example 2 -

Now, Example 2 is described. In Example 2, the speech features included in the present exemplary embodiment were used to train a machine learning model. A trained model was generated and speech data from examination subjects-excluding examination subjects from whom the training data was collected-was judged between the following two groups.

1. NYHA ≥ 2 and BNP ≥ 300
2. NYHA < 2 and BNP < 300

The entered speech data was not language-dependent. Speech data of two categories was used: (1) the sustained vowel sound "/a:/" and (2) the sound "pataka, pataka, ..." (repeated at least five times).

The results achieved an accuracy between the two groups of 81.97%, with the area under the curve (AUC) = 0.82.

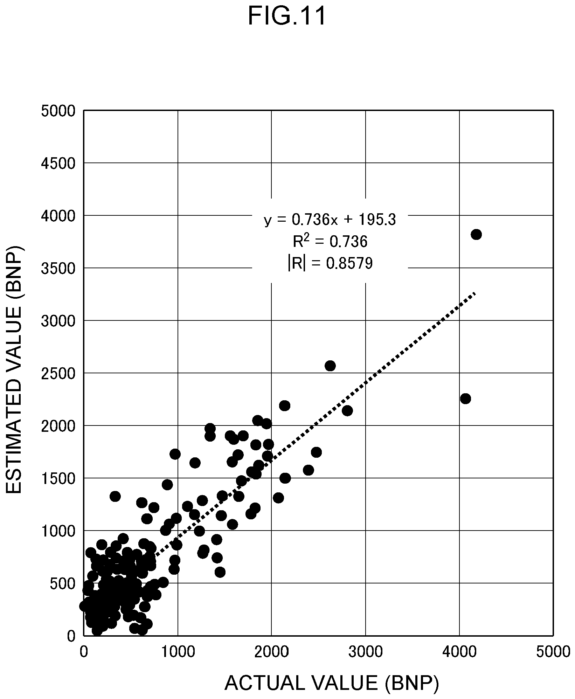

Fig. 11 shows results of using the speech features included in the present exemplary embodiment to compute BNP, which expresses a worsening of heart failure. Results for the regression line equation are as shown in Fig. 11.

The technology of the present disclosure is not limited by the exemplary embodiments described above; numerous modifications and applications are possible within a scope not departing from the gist of the invention.

In the exemplary embodiments described above, examples are described in which a degree of heart failure of a user is estimated as the cardiac condition of the user, but this is not limiting. For example, the cardiac condition of the user may be a cardiac load condition of the user, a pulmonary congestion condition of the user, a fluid retention condition of the user or the like. When estimating these conditions, corresponding scores are respectively specified. For example, a score relating to a cardiac load condition of a user may represent a level of cardiac load of the user, and a score relating to a pulmonary congestion or fluid retention condition of a user may represent a level of pulmonary congestion or fluid retention of the user.

As an example, the Description of the present Application describes exemplary embodiments in which a program is installed in advance, but the program may be stored and provided on a computer-readable recording medium.

The processing that, in the exemplary embodiments described above, is executed by a CPU reading software (a program) may be executed by various kinds of processor other than a CPU. Examples of processors in these cases include a PLD (programmable logic device) in which a circuit configuration can be modified after manufacturing, such as an FPGA (field-programmable gate array) or the like, a dedicated electronic circuit which is a processor with a circuit configuration that is specially designed to execute specific processing, such as an ASIC (application-specific integrated circuit) or the like, and so forth. A general-purpose graphics processing unit (GPGPU) may also be used as a processor. The processing may be executed by one of these various kinds of processors, and may be executed by a combination of two or more processors of the same or different kinds (for example, plural FPGAs, a combination of a CPU with an FPGA, or the like). Hardware structures of these various kinds of processors are, to be more specific, electronic circuits combining circuit components such as semiconductor components and the like.

In the exemplary embodiments described above, a mode is described in which the program is memorized in advance (installed) at the storage, but this is not limiting. The program may be provided in a mode that is recorded at a recording medium such as a CD-ROM (compact disc read-only memory), DVD-ROM (digital versatile disc read-only memory), USB (universal serial bus) memory or the like. Modes are also possible in which the program is downloaded from external equipment via a network.

The processes of the present exemplary embodiments may be configured by a computer, server or the like equipped with a general-purpose arithmetic processing unit and a memory device or the like, and the processes may be executed by a program. This program may be memorized at a memory device, may be recorded on a recording medium such as a magneto-optic disc, an optical disc, a semiconductor memory or the like, and may be provided through a network. Clearly, any other structural elements need not be implemented by a single computer, server or the like but may be distributed and realized at plural computers connected by a network.

All references, patent applications and technical specifications cited in the present specification are incorporated by reference into the present specification to the same extent as if the individual references, patent applications and technical specifications were specifically and individually recited as being incorporated by reference.

**Claims**

1.  An information processing device comprising:

    an acquisition section that acquires speech data that is time series data of speech spoken by a user;
    a computing section that computes state information representing a cardiac condition of the user based on the speech data acquired by the acquisition section; and
    an output section that outputs the state information computed by the computing section.

2.  The information processing device according to claim 1, wherein the computing section:

    generates an envelope of the speech data from the speech data and, by applying a Fourier transform to the envelope, acquires a Fourier transform result of the envelope;
    for respective combinations of a first frequency value and a second frequency value adjacent to the first frequency value in an analysis object frequency section of the Fourier transform result, computes differences between spectral powers of the first frequency values and spectral powers of the second frequency values; and
    computes an integration result integrating the differences in the analysis object frequency section, and computes the state information of the user based on the integration result.

3.  The information processing device according to claim 1, wherein the computing section:

    generates, from the speech data, a characteristic quantity representing at least one of a harmonics-to-noise ratio, a continuous vocalization duration, a proportion of intervals in plural utterances, a length of an utterance and a next utterance, a length of a speech interval, or a speaking speed; and
    computes the state information of the user based on the generated characteristic quantity.

4.  An information processing system comprising:

    a user terminal including a microphone; and
    the information processing device according to any one of claims 1 to 3,
    wherein:

        the user terminal transmits the speech data that is collected by the microphone to the information processing device,
        the acquisition section of the information processing device acquires the speech data transmitted from the user terminal,
a communications section of the information processing device transmits the state information computed by the computing section to the user terminal, and
the user terminal receives the state information transmitted from the information processing device.

5. The information processing system according to any one of claim 1 to claim 4, wherein the state information includes at least one of a degree of heart failure of the user, a cardiac load condition of the user, a pulmonary congestion condition of the user or a fluid retention condition of the user.

6. An information processing method including causing a computer to execute processing comprising:

   acquiring speech data that is time series data of speech spoken by a user;
   computing state information representing a cardiac condition of the user based on the acquired speech data; and
   outputting the computed state information.

7. An information processing program for causing a computer to execute processing comprising:

   acquiring speech data that is time series data of speech spoken by a user;
   computing state information representing a cardiac condition of the user based on the acquired speech data; and
   outputting the computed state information.

FIG.1

EP 4 534 021 A1

FIG.2A

# FIG.2B

FIG.2C

FIG.3

$VMI = |b-a| + |c-b| + |d-c| + ...$

FIG.4

# FIG.5

50

51

CPU

54

INPUT/OUTPUT
INTERFACE

55

READ/WRITE
SECTION

56

NETWORK
INTERFACE

57

MEMORY
SECTION

53

MEMORY

52

# FIG.6

START

ACQUIRE SPEECH DATA — S100

GENERATE ENVELOPE
OF SPEECH DATA — S102

APPLY FOURIER
TRANSFORM TO ENVELOPE — S104

SPECIFY ANALYSIS
OBJECT FREQUENCY SECTION — S106

COMPUTE SPECTRAL POWER DIFFERENCES
BETWEEN FIRST FREQUENCY VALUES
AND SECOND FREQUENCY VALUES — S108

COMPUTE INTEGRATION RESULT
INTEGRATING DIFFERENCES — S110

EXTRACT OTHER SPEECH
FEATURES FROM SPEECH DATA — S112

EXTRACT PLURAL SPEECH
FEATURES FROM REFERENCE DATA — S114

COMPUTE SCORE — S116

OUTPUT ESTIMATION RESULT — S118

END

FIG.7

# FIG.8

# FIG.9

FIG.10

EP 4 534 021 A1

FIG.11

# INTERNATIONAL SEARCH REPORT

| International application No. |
|---|
| **PCT/JP2022/022687** |

### A. CLASSIFICATION OF SUBJECT MATTER

***A61B 10/00***(2006.01)i
FI:   A61B10/00 K

According to International Patent Classification (IPC) or to both national classification and IPC

### B. FIELDS SEARCHED

Minimum documentation searched (classification system followed by classification symbols)

A61B10/00, A61B5/08-5/097

Documentation searched other than minimum documentation to the extent that such documents are included in the fields searched

Published examined utility model applications of Japan 1922-1996
Published unexamined utility model applications of Japan 1971-2022
Registered utility model specifications of Japan 1996-2022
Published registered utility model applications of Japan 1994-2022

Electronic data base consulted during the international search (name of data base and, where practicable, search terms used)

JSTPlus/JMEDPlus/JST7580 (JDreamIII)

### C. DOCUMENTS CONSIDERED TO BE RELEVANT

| Category* | Citation of document, with indication, where appropriate, of the relevant passages | Relevant to claim No. |
|---|---|---|
| X | MURTON, Olivia M. Acoustic speech analysis of patients with decompensated heart failure: A pilot study. The Journal of the Acoustical Society of America. vol. 142. no. 4. EL401-EL407<br>    in particular, 2. Methods, 3. Results | 1, 3, 6-7 |
| Y |  | 4-5 |
| A |  | 2 |
| A | JP 2008-284151 A (CHEST M I INC) 27 November 2008 (2008-11-27)<br>    paragraph [0006] | 1-7 |
| Y | JP 2020-507437 A (CARDIOKOL LTD) 12 March 2020 (2020-03-12)<br>    paragraphs [0008], [0217] | 4-5 |
| A | AMIR, Offer. Remote Speech Analysis in the Evaluation of Hospitalized Patients With Acute Decompensated Heart Failure. JACC: HEART FAILURE. January 2022, vol. 10, no. 1, pp. 41-49<br>    in particular, METHODS | 1-7 |

✓ Further documents are listed in the continuation of Box C.      ✓ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **16 June 2022** | **09 August 2022** |

| Name and mailing address of the ISA/JP | Authorized officer |
|---|---|
| **Japan Patent Office (ISA/JP)**<br>**3-4-3 Kasumigaseki, Chiyoda-ku, Tokyo 100-8915**<br>**Japan** | |
| | Telephone No. |

Form PCT/ISA/210 (second sheet) (January 2015)

## INTERNATIONAL SEARCH REPORT

International application No.

**PCT/JP2022/022687**

### C.    DOCUMENTS CONSIDERED TO BE RELEVANT

| Category* | Citation of document, with indication, where appropriate, of the relevant passages | Relevant to claim No. |
|---|---|---|
| A | REDDY, M. Kiran. The automatic detection of heart failure using speech signals. Computer Speech & Language. 27 February 2021, vol. 69, 101205<br>in particular, 2. The proposed method | 1-7 |

Form PCT/ISA/210 (second sheet) (January 2015)

## INTERNATIONAL SEARCH REPORT
### Information on patent family members

| International application No. |
|---|
| **PCT/JP2022/022687** |

| Patent document cited in search report | | | Publication date (day/month/year) | Patent family member(s) | Publication date (day/month/year) |
|---|---|---|---|---|---|
| JP | 2008-284151 | A | 27 November 2008 | (Family: none) | |
| JP | 2020-507437 | A | 12 March 2020 | US 2019/0362740 A1<br>paragraphs [0019]-[0022], [0311]<br>WO 2018/146690 A1<br>EP 3580754 A1<br>KR 10-2019-0113968 A<br>CN 110494916 A | |

Form PCT/ISA/210 (patent family annex) (January 2015)

**EP 4 534 021 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 2020013296 A

**Non-patent literature cited in the description**

- Harmonic to Noise Ratio Measurement - Selection of Window and Length. *Procedia Computer Science*, 2018, vol. 138, 280-285